# EUROPEAN PATENT APPLICATION

(11) **EP 4 834 311 A1**
(43) Date of publication of application: **04.11.2026**
(21) Application number: 24912439.7
(22) Date of filing: 13.12.2024
(51) Int. Cl.: A61K 8/06, A61K 8/29, A61K 8/86, A61Q 1/02

(54) **OIL-IN-WATER TYPE EMULSION COSMETIC**

(30) Priority: 28.12.2023 JP 2023223656
(71) Applicant: Shiseido Company, Ltd., Chuo-ku Tokyo 104-0061 (JP)
(72) Inventor: KATORI, Takahiro, Tokyo 104-0061 (JP); SUN, Yudi, Tokyo 104-0061 (JP)
(74) Representative: Ter Meer und Partner mbB
(86) International application number: PCT/JP2024/044174
(87) International publication number: WO 2025/142550

(57) **Abstract**

An oil-in-water emulsion cosmetic includes (A) an amphiphile and (B) hydrophobic-treated metal oxide fine particles that have been subjected to a hydrophobic treatment using monoalkyl phosphate. The (A) amphiphile includes one or more selected from the group consisting of alkylene oxide derivatives represented by general formulae (1) and (2) below, 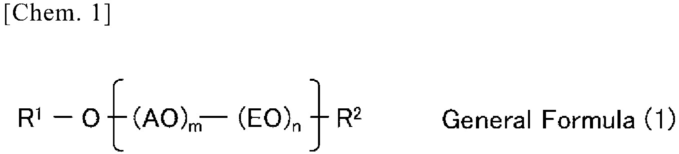 (in the general formula (1), AO is a C3-4 oxyalkylene group, EO is an oxyethylene group, R¹ and R² are each independently a C1-4 hydrocarbon group or a hydrogen atom, and m and n are integers of 1≤ m ≤ 70, and 1 ≤ n ≤ 70, respectively) 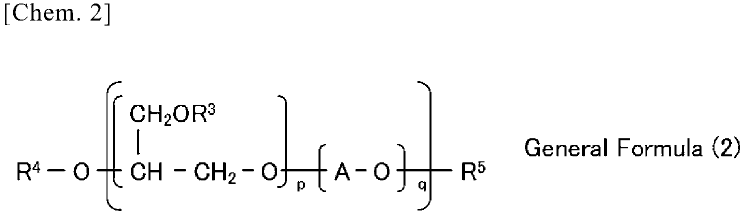 (in the general formula (2), R³, R⁴, and R⁵ are each independently a hydrogen atom or a C1-4 alkyl group, A is a C2-4 alkylene group, and p and q are each independently an integer of 1 to 50, where a polymer represented by the general formula (2) has a number average molecular weight of 10,000 or less, and an IOB value of 0.4 to 1.8).

## Description

### TECHNICAL FIELD

The present invention relates to oil-in-water emulsion cosmetics.

### BACKGROUND ART

In recent years, as various materials in cosmetics, use of materials that are highly safe for human bodies, such as materials that conform to the EU standards, has been desired in several countries.

Among materials to be included in cosmetics, surface-treated metal oxide is often used as an ultraviolet scattering agent in cosmetics, such as sunscreens, foundations, and the like. For example, Patent Document 1 discloses, as a material capable of blocking ultraviolet rays, zinc oxide particles, which are surface-treated with octyltriethoxysilane, excel in color and a feel during use, and can be suitably used in cosmetics.

### CITATION LIST

### PATENT DOCUMENT

Patent Document 1: Japanese Unexamined Patent Application Publication No. 2023-162678

### SUMMARY OF THE INVENTION

### TECHNICAL PROBLEM

However, there is a demand for use of a surface-treating agent that is safer than octyltriethoxysilane that is used as a surface-treating agent for zinc oxide particles in Patent Document 1.

However, it is difficult to perform a surface treatment by replacing the surface-treating agent used for conventional ultraviolet scattering agents, such as octyltriethoxysilane, with a surface-treating agent that is considered to be safer. Use of such a surface-treating agent may lead to an insufficient surface treatment of zinc oxide, titanium oxide, or the like constituting cores of the ultraviolet scattering agent, and therefore emulsion stability tends to become poor when such an ultraviolet scattering agent is blended in a cosmetic or the like.

One aspect of the present invention aims to provide an oil-in-water emulsion cosmetic that is highly safe and has favorable emulsion stability.

### SOLUTION TO THE PROBLEM

In order to solve the above problems, one aspect of the present invention provides an oil-in-water emulsion cosmetic, which includes (A) an amphiphile and (B) hydrophobic-treated metal oxide fine particles that have been subjected to a hydrophobic treatment using monoalkyl phosphate, wherein the (A) amphiphile includes one or more selected from the group consisting of alkylene oxide derivatives represented by a general formula (1) below and a general formula (2) below. 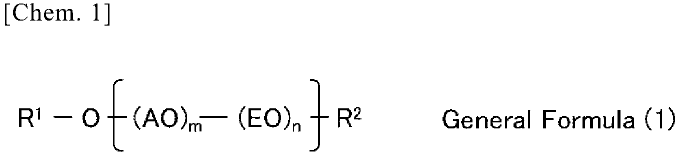 (In the general formula (1), AO is an oxyalkylene group with 3 to 4 carbon atoms, EO is an oxyethylene group, R¹ and R² are each independently a hydrocarbon group with 1 to 4 carbon atoms or a hydrogen atom, and m and n are integers of 1≤ m ≤ 70, and 1 ≤ n ≤ 70, respectively.) 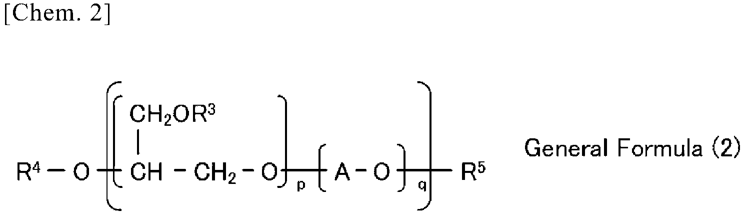 (In the general formula (2), R³, R⁴, and R⁵ are each independently a hydrogen atom or an alkyl group with 1 to 4 carbon atoms, A is an alkylene group with 2 to 4 carbon atoms, and p and q are each independently an integer of 1 to 50, where a polymer represented by the general formula (2) has a number average molecular weight of 10,000 or less and an IOB value of 0.4 to 1.8.)

### EFFECTS OF THE INVENTION

According to one aspect of the present invention, there can be provided an oil-in-water emulsion cosmetic that is highly safe and has favorable emulsion stability.

### DETAILED DESCRIPTION OF THE EMBODIMENTS

Hereinafter, embodiments of the present invention will be described in detail.

### (Oil-in-water emulsion cosmetic)

The oil-in-water emulsion cosmetic of the present invention includes (A) an amphiphile and (B) hydrophobic-treated metal oxide fine particles that have been subjected to a hydrophobic treatment using monoalkyl phosphate. The (A) amphiphile includes one or more selected from the group consisting of alkylene oxide derivatives represented by a general formula (1) below and a general formula (2) below. 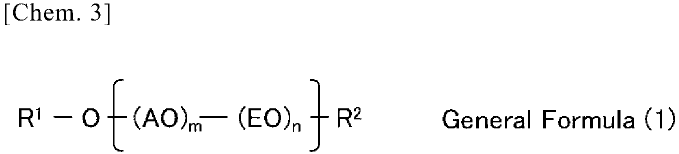 (In the general formula (1), AO is an oxyalkylene group with 3 to 4 carbon atoms, EO is an oxyethylene group, R¹ and R² are each independently a hydrocarbon group with 1 to 4 carbon atoms or a hydrogen atom, and m and n are integers of 1≤ m ≤ 70, and 1 ≤ n ≤ 70, respectively.) 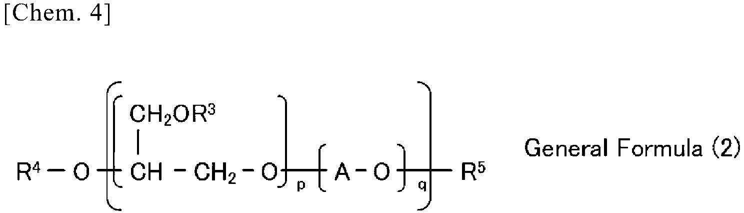 (In the general formula (2), R³, R⁴, and R⁵ are each independently a hydrogen atom or an alkyl group with 1 to 4 carbon atoms, A is an alkylene group with 2 to 4 carbon atoms, and p and q are each independently an integer of 1 to 50, where a polymer represented by the general formula (2) has a number average molecular weight of 10,000 or less and an IOB value of 0.4 to 1.8.)

According to the present invention, there can be provided an oil-in-water emulsion cosmetic that is highly safe and has favorable emulsion stability.

### <(A) Amphiphile>

The oil-in-water emulsion cosmetic of the present invention includes, as the (A) component, one or more selected from the group consisting of alkylene oxide derivatives represented by the following general formula (1) and the following general formula (2), which are amphiphiles. 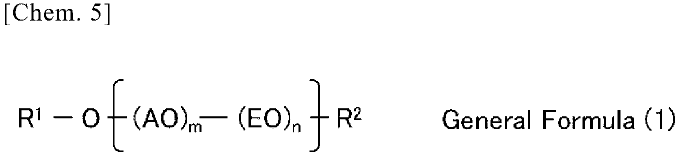 (In the general formula (1), AO is an oxyalkylene group with 3 to 4 carbon atoms, EO is an oxyethylene group, R¹ and R² are each independently a hydrocarbon group with 1 to 4 carbon atoms or a hydrogen atom, and m and n are integers of 1≤ m ≤ 70, and 1 ≤ n ≤ 70, respectively.) 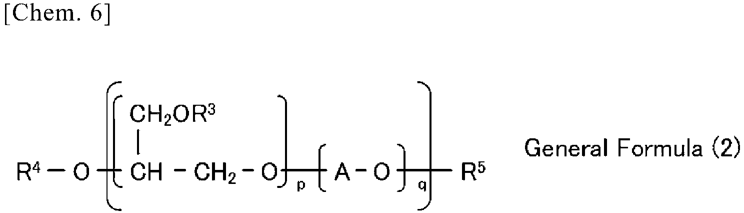 (In the general formula (2), R³, R⁴, and R⁵ are each independently a hydrogen atom or an alkyl group with 1 to 4 carbon atoms, A is an alkylene group with 2 to 4 carbon atoms, and p and q are each independently an integer of 1 to 50, where a polymer represented by the general formula (2) has a number average molecular weight of 10,000 or less, and an IOB value of 0.4 to 1.8.)

Since the oil-in-water emulsion cosmetic of the present invention includes the amphiphile, the cosmetic has a moisturizing effect and excels in a feel during use, particularly excels in smoothness and no sticky feel.

The amount of the amphiphile in the oil-in-water emulsion cosmetic of the present invention is preferably 0.1 percent by mass or greater and 10 percent by mass or less, more preferably 0.5 percent by mass or greater and 7 percent by mass or less, and yet more preferably 1 percent by mass or greater and 5 percent by mass or less.

When the amount of the amphiphile in the oil-in-water emulsion cosmetic is 0.1 percent by mass or greater, the oil-in-water emulsion cosmetic has a sufficient moisturizing effect. When the amount of the amphiphile in the oil-in-water emulsion cosmetic is 10 percent by mass or less, stickiness is hardly felt after use.

### <<Alkylene oxide derivative represented by general formula (1)>>

In the alkylene oxide derivative represented by the following general formula (1), AO is an oxyalkylene group with 3 to 4 carbon atoms, and EO is an oxyethylene group.

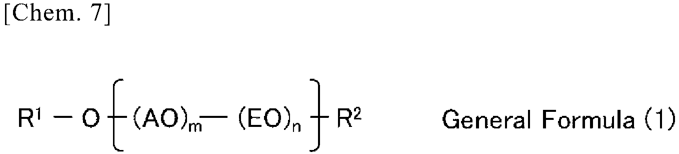

Since the oil-in-water emulsion cosmetic includes the alkylene oxide derivative represented by the general formula (1), the cosmetic has a moisturizing effect and excels in a feel during use, particularly excels in smoothness and no stickiness.

Specific examples of the oxyalkylene group with 3 to 4 carbon atoms, which is represented by AO, include an oxypropylene group, an oxybutylene group, an oxyisobutylene group, an oxytrimethylene group, an oxytetramethylene group, and the like, with an oxypropylene group, an oxybutylene group, or the like being preferable.

In the general formula (1), m is an average number of moles of AO added per molecule, and is 1 ≤ m ≤ 70, preferably 1 ≤ m ≤ 30, and more preferably 1 ≤ m ≤ 20.

In the general formula (1), n is an average number of moles of EO added per molecule, and is 1 ≤ n ≤ 70, preferably 1 ≤ n ≤ 30, and more preferably 1 ≤ n ≤ 20.

When each of the number of moles of the oxyalkylene group with 3 to 4 carbon atoms and the number of moles of the oxyethylene group is 1 or greater, a moist feel can be sufficiently realized. When each of the number of moles of the oxyalkylene group with 3 to 4 carbon atoms and the number of moles of the oxyethylene group is 70 or less, a smooth feel is sufficiently ensured without causing a sticky feel. When the value of m + n is too large, the resultant oil-in-water emulsion cosmetic may leave a sticky feel. Therefore, m + n is preferably 8 to 100, preferably 8 to 40, more preferably 8 to 25 or less, and yet more preferably 8 to 20 or less.

The order of adding AO and EO in the alkylene oxide derivative represented by the general formula (1) is not particularly limited. AO and EO may be added in a block form to form a block copolymer, or may be randomly added to form a random copolymer, and a random copolymer of AO and EO is preferable. The block copolymer includes a copolymer including three or more blocks, as well as a copolymer including two blocks.

The molecular weight of the alkylene oxide derivative represented by the general formula (1) is preferably 100 to 10,000, more preferably 200 to 5,000, and yet more preferably 300 to 2,000. In addition, a ratio [EO/(AO + EO)] of EO to a sum of AO and EO per molecule is preferably within the range of 20 to 80 percent by mass. When the ratio [EO/(AO + EO)] of EO is 20 percent by mass or greater, the resultant oil-in-water emulsion cosmetic leaves a moist feel. When the ratio [EO/(AO + EO)] of EO is 80 percent by mass or less, the resultant oil-in-water emulsion cosmetic leaves a smooth feel.

In the general formula (1), R¹ and R² are each independently a hydrocarbon group with 1 to 4 carbon atoms or a hydrogen atom. Examples of the hydrocarbon group include an alkyl group, such as a methyl group, an ethyl group, an n-propyl group, an isopropyl group, an n-butyl group, a sec-butyl group, a tert-butyl group, and the like. The hydrocarbon group is preferably a methyl group or an ethyl group.

R¹ and R² in a molecule of the alkylene oxide derivative represented by the general formula (1) may be the same hydrocarbon groups, a mixture of a hydrocarbon group and a hydrogen atom, a mixture of hydrocarbon groups having mutually different numbers of carbon atoms. As the abundance of a hydrocarbon group and a hydrogen atom as each of R¹ and R², a ratio (Y/X) of the number of the hydrogen atom (Y) to the number of the hydrocarbon group (X) is preferably 0.15 or less, and more preferably 0.06 or less. When the ratio (Y/X) is 0.15 or less, the resultant oil-in-water emulsion cosmetic does not leave a sticky feel.

Specific examples of the alkylene oxide derivative represented by the general formula (1) preferably used in the present invention include, but are not limited to, for example, the following.

PEG/PPG-9/2 dimethyl ether
PEG/PPG-17/4 dimethyl ether
PEG/PPG-14/7 dimethyl ether
PEG/PPG-11/9 dimethyl ether
PEG/PPG-55/28 dimethyl ether
PEG/PPG-36/41 dimethyl ether
PEG/PPG-6/3 dimethyl ether
PEG/PPG-8/4 dimethyl ether
PEG/PPG-6/11 dimethyl ether
PEG/PPG-14/27 dimethyl ether

The alkylene oxide derivative is more likely to have an excellent effect of improving ultraviolet protection performance due to heat, as the molecular weight of the alkylene oxide derivative is relatively smaller. Accordingly, among the above-listed alkylene oxide derivatives represented by the general formula (1), PEG/PPG-9/2 dimethyl ether is particularly preferable.

The alkylene oxide derivative represented by the general formula (1) can be produced by a known method. For example, a compound including a hydroxyl group is subjected to addition polymerization with ethylene oxide and alkylene oxide with 3 to 4 carbon atoms, and then a resultant polymer is reacted with halogenated alkyl through an esterification reaction in the presence of an alkali catalyst, thereby producing the alkylene oxide derivative represented by the general formula (1).

The amount of the alkylene oxide derivative represented by the general formula (1) in the present invention is not particularly limited, and is preferably 0.1 to 10 percent by mass, more preferably 0.5 to 7 percent by mass, and yet more preferably 1 to 5 percent by mass. When the amount of the alkylene oxide derivative represented by the general formula (1) is 0.1 percent by mass or greater, the oil-in-water emulsion cosmetic has a sufficient moisturizing effect. When the amount of the alkylene oxide derivative represented by the general formula (1) is 10 percent by mass or less, a sticky feel is hardly felt after use.

The alkylene oxide derivative represented by the general formula (1) has a moisturizing effect. In addition, when another moisturizing agent, such as glycerin or the like, is further blended, an effect of minimizing stickiness is observed. Examples of the moisturizing agent that can be used in combination include polyethylene glycol, propylene glycol, glycerin, 1,3-butylene glycol, xylitol, sorbitol, maltitol, chondroitin sulfate, hyaluronic acid, mucoitin sulfate, caronic acid, atelocollagen, cholesteryl-12-hydroxystearate, sodium lactate, bile salts, dl-pyrrolidone carboxylate, short-chain soluble collagen, diglycerin (EO) PO adducts (diglycerin ethylene oxide·propylene oxide adducts), Rosa roxburghii extracts, Achillea millefolium extracts, Melilotus extracts, and the like.

The alkylene oxide derivative represented by the general formula (1) also has an effect of facilitating stratum corneum penetration of another moisturizing agent. Therefore, when the alkylene oxide derivative represented by the general formula (1) and another moisturizing agent are used in combination in the oil-in-water emulsion cosmetic of the present invention, an extremely high moisturizing effect can be obtained due to the synergistic effect of the alkylene oxide derivative represented by the general formula (1) and another moisturizing agent. Examples of such moisturizing agent include those listed above. In particular, the moisturizing agent used in combination is preferably glycerin or xylitol.

The amount of another moisturizing agent in the present invention is not particularly limited, and is preferably 0.001 to 20 percent by mass, and more preferably 0.1 to 10 percent by mass. In addition, the alkylene oxide derivative represented by the general formula (1) has an effect of facilitating stratum corneum penetration of a hydrophilic drug other than the moisturizing agent, for example, a whitening agent such as arbutin.

### <<Alkylene oxide derivative represented by general formula (2)>>

In the alkylene oxide derivative represented by the following general formula (2), R³, R⁴, and R⁵ are each independently a hydrogen atom or an alkyl group with 1 to 4 carbon atoms, A is an alkylene group with 2 to 4 carbon atoms, and p and q are each independently an integer of 1 to 50. In addition, a polymer represented by the following general formula (2) has a number average molecular weight of 10,000 or less and an IOB value of 0.4 to 1.8.

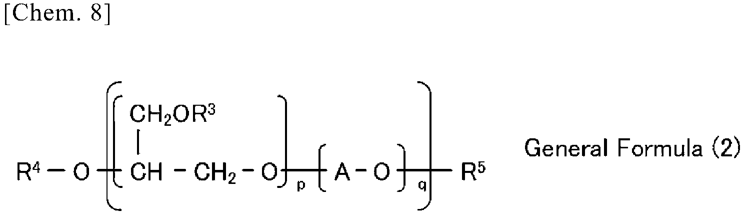

Since the oil-in-water emulsion cosmetic includes the alkylene oxide derivative represented by the general formula (2), the cosmetic has a moisturizing effect and excels in a feel during use, particularly excels in smoothness and no sticky feel.

When R³, R⁴, and R⁵ of the general formula (2) are alkyl groups with 1 to 4 carbon atoms, examples of R³, R⁴, and R⁵ include a methyl group, an ethyl group, an n-propyl group, an isopropyl group, an n-butyl group, a sec-butyl group, a tert-butyl group, and the like. In addition, these alkyl groups may further include a substituent, as long as the effects of the present invention are not impaired. The substituent is not particularly limited. Examples of the substituent include a halogeno group and the like.

In the general formula (2), R³, R⁴, and R⁵ may each independently be a hydrogen atom, a methyl group, an ethyl group, an n-propyl group, an isopropyl group, an n-butyl group, a sec-butyl group, or a tert-butyl group.

In the general formula (2), R³ is preferably a hydrogen atom, a methyl group, or an ethyl group. In addition, in the case where p is 2 or greater, specifically, in the case where there are two or more R³, the two or more R³ may be the same or different. In particular, at least some of R³ is preferably a methyl group. In the case where some of the two or more R³ is a methyl group, it is particularly preferable from the viewpoint of improvement in a feel during use.

In the general formula (2), R³ may be a mixture of a hydrogen atom and an alkyl group with 1 to 4 carbon atoms. Specifically, some of R³ may be a hydrogen atom, and some of the rest of R³ may be an alkyl group with 1 to 4 carbon atoms. Preferably, some of R³ is a hydrogen atom, and some of the rest of R³ is a methyl group.

According to one embodiment of the present invention, in the general formula (2), R⁴ is preferably a hydrogen atom, a methyl group, or an ethyl group, and in particular, more preferably a methyl group.

According to one embodiment of the present invention, in the general formula (2), R⁴ may be a mixture of a hydrogen atom and an alkyl group with 1 to 4 carbon atoms. Specifically, some of R⁴ may be a hydrogen atom, and some of the rest of R⁴ may be an alkyl group with 1 to 4 carbon atoms. Preferably, some of R⁴ is a hydrogen atom, and some of the rest of R⁴ is a methyl group. In addition, in the case where there are two or more R³ and two or more R⁴ and at least some of them are methyl groups, a ratio (CH₃/H) of a methyl group to hydrogens as R³ and R⁴ is not particularly limited, and may be, for example, 0.05 or greater, 0.10 or greater, 0.15 or greater, 0.20 or greater, 0.25 or greater, 0.30 or greater, or 0.35 or greater, and may be 0.99 or less, 0.90 or less, 0.80 or less, 0.70 or less, or 0.60 or less.

According to one embodiment of the present invention, in the general formula (2), R⁵ is preferably a hydrogen atom, a methyl group, or an ethyl group, and in particular, more preferably a methyl group. In addition, in the case where there are two or more R³ and at least some of the two or more R³ is a methyl group, a ratio of the methyl group to hydrogens (CH₃/H) as R³, R⁴, and R⁵, in addition to the above-described R³ and R⁴, is not particularly limited, and may be, for example, 0.05 or greater, 0.10 or greater, 0.15 or greater, 0.20 or greater, 0.25 or greater, 0.30 or greater, or 0.35 or greater, and may be 0.99 or less, 0.90 or less, 0.80 or less, 0.70 or less, or 0.60 or less.

According to one embodiment of the present invention, in the general formula (2), at least either R⁴ or R⁵ is preferably a methyl group.

In the general formula (2), A is an alkylene group with 2 to 4 carbon atoms. More specifically, A is, for example, an ethylene group, a propylene group, a butylene group, an isobutylene group, or the like, but is not limited to the foregoing. In particular, A is preferably represented by the following general formula (3).

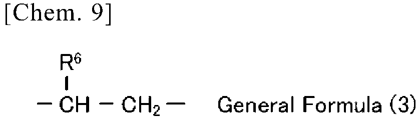

In the general formula (3), R⁶ is an alkyl group with 1 or 2 carbon atoms. More specifically, R⁶ may be a methyl group or an ethyl group.

In the general formula (2), p and q each represent an average number of moles of a respective constituent unit, and p and q are each independently an integer of 1 to 50. Specifically, p and q may be 1 or greater, 2 or greater, 3 or greater, 4 or greater, 5 or greater, 6 or greater, 7 or greater, 8 or greater, 9 or greater, 10 or greater, 15 or greater, or 20 or greater, and may be 50 or less, 45 or less, 40 or less, 35 or less, 34 or less, 32 or less, 30 or less, 28 or less, 26 or less, 25 or less, 24 or less, 22 or less, 20 or less, 18 or less, 16 or less, 15 or less, 14 or less, 12 or less, 10 or less, or 5 or less.

In addition, in the general formula (2), p is preferably 1 or greater and 25 or less, 1 or greater and 20 or less, or 1 or greater and 14 or less, and more preferably 2 or greater and 20 or less, 2 or greater and 15 or less, or 2 or greater and 5 or less. In addition, in the general formula (2), q is preferably 2 or greater and 40 or less, 2 or greater and 35 or less, or 2 or greater and 34 or less, and more preferably 6 or greater and 35 or less, or 6 or greater and 12 or less.

According to one embodiment of the present invention, in the general formula (2), p + q may be 4 or greater, 5 or greater, 6 or greater, 7 or greater, 8 or greater, 9 or greater, 10 or greater, 11 or greater, 12 or greater, 13 or greater, 14 or greater, 15 or greater, or 17 or greater, and may be 55 or less, 53 or less, 50 or less, 45 or less, 42 or less, 40 or less, 35 or less, 30 or less, 25 or less, 20 or less, 15 or less, or 12 or less.

According to one embodiment of the present invention, in the general formula (2), p : q may be 1 : 10 to 10 : 1, 1 : 6 to 6 : 1, or 1 : 5 to 5 : 1.

The number average molecular weight of the polymer represented by the general formula (2) is 10,000 or less. More specifically, the number average molecular weight of the polymer represented by the general formula (2) may be 10,000 or less, 5,000 or less, 3,500 or less, 3,000 or less, 2,800 or less, 2,600 or less, 2,500 or less, 2,000 or less, 1,500 or less, 1,400 or less, 1,300 or less, 1,200 or less, 1,100 or less, 1,000 or less, 900 or less, 800 or less, 700 or less, or 600 or less, and may be 130 or greater, 150 or greater, 200 or greater, 250 or greater, 300 or greater, 350 or greater, 400 or greater, 450 or greater, or 500 or greater.

In addition, the number average molecular weight of the polymer represented by the general formula (2) is preferably 300 or greater and 3,500 or less, and more preferably 500 or greater and 3,500 or less, or 500 or greater and 1,200 or less.

The IOB value of the polymer represented by the general formula (2) is 0.4 to 1.8. More specifically, the IOB value of the polymer represented by the general formula (2) may be, for example, 0.4 or greater, 0.5 or greater, 0.6 or greater, 0.7 or greater, 0.8 or greater, or 0.9 or greater, and may be 1.8 or less, 1.6 or less, 1.4 or less, 1.2 or less, or 1.0 or less. Moreover, the IOB value of the polymer represented by the general formula (2) is preferably 0.7 or greater and 1.2 or less.

Here, the IOB value is an abbreviation of Inorganic/Organic Balance (inorganic/organic ratio), and a value representing a ratio of an inorganic value to an organic value, which is an index representing a degree of polarity of an organic compound. Specifically, the IOB value is represented by the IOB value = an inorganic value/an organic value.

The "inorganic value" and the "organic value" are each set to an "inorganic value" and an "organic value" according to various atoms or functional groups, for example, the "organic value" being 20 for one carbon atom in a molecule, and the "inorganic value" being 100 for one hydroxyl group in the molecule. An IOB value of an organic compound can be calculated by integrating the "inorganic values" and the "organic values" of all atoms and functional groups in the organic compound (see, for example, Yoshio KODA, "Organic Conceptual Diagram-Foundation and Application-", p. 11-17, published by SANKYO SHUPPAN Co., Ltd., 1984).

The polymer represented by the general formula (2) may be a block copolymer or a random copolymer.

A method for producing the polymer represented by the general formula (2) is not particularly limited. For example, the production may be performed by addition polymerization of epoxide corresponding to each constituent unit. In addition, the addition polymerization may be block polymerization or random polymerization.

In addition, the method for producing the polymer represented by the general formula (2) may further include a further reaction of the polymer obtained by the above addition polymerization with halogenated alkyl. In a molecule of the polymer obtained through the reaction with the halogenated alkyl, the hydroxyl group is capped with the alkyl group, and as a result, hydrophobicity of the entire polymer can be adjusted as desired. The capping rate of the hydroxyl group with the alkyl group in the molecule of the polymer represented by the general formula (2) may be, for example, 20% or greater, 30% or greater, 35% or greater, 40% or greater, 45% or greater, 50% or greater, or 55% or greater, and may be 60% or less, 59% or less, 58% or less, 57% or less, 56% or less, or 55% or less.

The polymer represented by the general formula (2) has both high water solubility and high lipid solubility.

The solubility of the polymer represented by the general formula (2) in water may be, for example, 5 percent by mass or greater, 10 percent by mass or greater, 20 percent by mass or greater, 30 percent by mass or greater, 40 percent by mass or greater, or 50 percent by mass or greater.

As an index for the lipid solubility of the polymer represented by the general formula (2), for example, solubility in oil can be used. The solubility of the polymer represented by the general formula (2) in olive oil may be, for example, 0.01 percent by mass or greater, 0.05 percent by mass or greater, 0.10 percent by mass or greater, 0.50 percent by mass or greater, or 1.00 percent by mass or greater. The upper limit value of the solubility of the polymer represented by the general formula (2) in the olive oil is not particularly limited, and may be, for example, 10 percent by mass or less.

The amount of the alkylene oxide derivative represented by the general formula (2) in the present invention is not particularly limited, and is preferably 0.1 percent by mass or greater and 10 percent by mass or less, more preferably 0.5 percent by mass or greater and 7 percent by mass or less, and yet more preferably 1 percent by mass or greater and 5 percent by mass or less. When the amount of the alkylene oxide derivative represented by the general formula (2) is 0.1 percent by mass or greater, the oil-in-water emulsion cosmetic has a sufficient moisturizing effect. When the amount of the alkylene oxide derivative represented by the general formula (2) is 10 percent by mass or less, a sticky feel is hardly felt after use.

### <(B) Hydrophobic-treated metal oxide fine particles subjected to hydrophobic treatment using monoalkyl phosphate>

The oil-in-water emulsion cosmetic of the present invention includes, as the (B) component, hydrophobic-treated metal oxide fine particles that have been subjected to a hydrophobic treatment using monoalkyl phosphate. The hydrophobic-treated metal oxide fine particles that have been subjected to a hydrophobic treatment using monoalkyl phosphate are metal oxide fine particles serving as cores, which have been subjected to a hydrophobic treatment using monoalkyl phosphate, and function as an ultraviolet scattering agent in the oil-in-water emulsion composition of the present invention.

Examples of the hydrophobic-treated metal oxide fine particles that have been subjected to a hydrophobic treatment using monoalkyl phosphate, which can be used in the oil-in-water emulsion cosmetic of the present invention, include fine particles of metal oxide, such as titanium oxide, zinc oxide, iron oxide, cerium oxide, tungsten oxide, or the like, surfaces of which are processed by a hydrophobic surface treatment using monoalkyl phosphate.

Examples of the monoalkyl phosphate include cetyl phosphate, ethyl phosphate, octyl phosphate, stearyl phosphate, dodecyl phosphate, and the like. The monoalkyl phosphate preferably includes cetyl phosphate.

The (B) hydrophobic-treated metal oxide fine particles that have been subjected to a hydrophobic treatment using monoalkyl phosphate, serving as the ultraviolet scattering agent, may be in the form of particles. In such a case, an average primary particle diameter is not particularly limited, and may be, for example, 5 nm to 200 nm, 8 nm to 100 nm, 10 to 50 nm, or 12 to 30 nm. For example, the "average primary particle diameter" may be determined as a diameter of an equivalent circle of a projected area of a primary particle in a SEM image.

A silica coating film is preferably disposed on each of the hydrophobic-treated metal oxide fine particles that have been subjected to a hydrophobic treatment using monoalkyl phosphate. Specific example of such hydrophobic-treated metal oxide fine particles include metal oxide fine particles of titanium oxide, zinc oxide, iron oxide, cerium oxide, tungsten oxide, or the like, which are surface-coated with a silica coating film, and have been subjected to a hydrophobic surface treatment using monoalkyl phosphate.

In the case where the silica coating film is disposed on each of the metal oxide fine particles, the silica coating film is preferably formed as one or more layers, more preferably two or more layers, deposited over the entire surface of each of the metal oxide fine particles.

When the metal oxide fine particles are each coated with the silica coating film, the surface of the metal oxide is covered with silica, and therefore a catalytic activity of the metal can be inhibited, desirable stability is achieved, discoloration can be inhibited, and a texture during use can be improved. When two or more layers of the silica coating film are deposited, the above effects can be further enhanced.

The amount of the (B) hydrophobic-treated metal oxide fine particles that have been subjected to a hydrophobic treatment using monoalkyl phosphate in the present invention is not particularly limited, and is preferably 1 percent by mass or greater and 10 percent by mass or less, more preferably 2 percent by mass or greater and 7 percent by mass or less, and yet more preferably 3 percent by mass or greater and 5 percent by mass or less. When the amount of the (B) hydrophobic-treated metal oxide fine particles is 1 percent by mass or greater, an effect as the ultraviolet scattering agent can be sufficiently ensured. When the amount of the (B) hydrophobic-treated metal oxide fine particles is 10 percent by mass or less, a favorable texture during use can be obtained.

### <(C) Active agent>

The oil-in-water emulsion cosmetic of the present invention may further include an active agent as the (C) component. For example, a surfactant or the like can be included.

Since the amphiphile has a property of being compatible with both water and oil, the amphiphile is easily detached from the interface, and an emulsion generally becomes unstable when the amphiphile is used together with a surfactant. However, although the oil-in-water emulsion cosmetic of the present invention includes the amphiphile, the oil-in-water emulsion cosmetic of the present invention surprisingly does not cause demulsification, and is stably emulsified.

Examples of the surfactant included in the oil-in-water emulsion cosmetic of the present invention as the (C) component include polyoxyethylene hydrogenated castor oil and the like. The polyoxyethylene hydrogenated castor oil is preferably included.

The polyoxyethylene hydrogenated castor oil increases hydrophilicity, which improves emulsion stability, as the average number of moles of ethylene oxide added increases. Therefore, in the case where the (C) component is the polyoxyethylene hydrogenated castor oil, the average number of moles of ethylene oxide added in the polyoxyethylene hydrogenated castor oil in the present invention is preferably 40 moles or greater, and more preferably 50 moles or greater and 100 moles or less. When the average number of moles of ethylene oxide added in the polyoxyethylene hydrogenated castor oil is 40 moles or greater, a certain degree of hydrophilicity is imparted, and therefore hydrophilicity can be easily ensured so that the polyoxyethylene hydrogenated castor oil is suitable for oil-in-water emulsification, and emulsion stability is easily improved. When the average number of moles of ethylene oxide added in the polyoxyethylene hydrogenated castor oil is 100 moles or less, the polyoxyethylene hydrogenated castor oil has low stickiness, and has favorable usability.

The amount of the (C) active agent in the oil-in-water emulsion cosmetic of the present invention is preferably 0.1 to 10 percent by mass or less, more preferably 0.5 to 5 percent by mass or less, and yet more preferably 0.5 to 3 percent by mass or less. When the amount of the (C) active agent is 0.1 percent by mass or greater, emulsion stability can be sufficiently improved. When the amount of the (C) active agent is 10 percent by mass or less, a favorable texture during use can be obtained.

Examples of the polyoxyethylene hydrogenated castor oil suitable for the oil-in-water emulsion cosmetic of the present invention includes PEG-40 hydrogenated castor oil, PEG-50 hydrogenated castor oil, PEG-60 hydrogenated castor oil, PEG-80 hydrogenated castor oil, PEG-100 hydrogenated castor oil, and the like.

### <Other components>

The oil-in-water emulsion cosmetic of the present invention can be appropriately include, as components other than the above, for example, powder components, liquid oils and fats, solid oils and fats, wax, hydrocarbon oil, higher fatty acids, higher alcohols, synthetic ester oil, silicone oil, silicones, anionic surfactants, cationic surfactants, amphoteric surfactants, nonionic surfactants, active agents other than the surfactants, water-soluble polymers, thickening agents, ultraviolet-ray absorbers, moisturizing agents, sequestering agents, lower alcohols, polyhydric alcohols, saccharides, amino acids, organic amines, polymer emulsions, neutralizing agents, pH adjusters, vitamins, antioxidants, antioxidation auxiliary agents, stabilizers, fragrances, water, and the like, as necessary, and can be produced by a common procedure according to an intended dosage form. Specific components that can be blended are listed below.

Examples of the powder components include: inorganic powders (e.g., talc, kaolin, mica, sericite (sericite), muscovite, phlogopite, synthetic mica, lepidolite, biotite, vermiculite, magnesium carbonate, calcium carbonate, aluminum silicate, barium silicate, calcium silicate, magnesium silicate, strontium silicate, metal tungstate, magnesium, silica, zeolite, barium sulfate, calcined calcium sulfate (calcined gypsum), calcium phosphate, fluoroapatite, hydroxyapatite, ceramic powders, metal soaps (e.g., zinc myristate, calcium palmitate, and aluminum stearate), boron nitride, etc.); organic powders (e.g., polyamide resin powders (nylon powders), polyethylene powders, polymethyl methacrylate powders, polystyrene powders, styrene-acrylic acid copolymer resin powders, benzoguanamine resin powders, polytetrafluoroethylene powders, cellulose powders, etc.); inorganic white pigments (e.g., titanium dioxide, zinc oxide, etc.); inorganic red pigments (e.g., iron oxide (red iron oxide), iron titanate, etc.); inorganic brown pigments (e.g., γ-iron oxide, etc.); inorganic yellow pigments (e.g., yellow iron oxide, yellow ocher, etc.); inorganic black pigments (e.g., black iron oxide, lower titanium oxide, etc.); inorganic purple pigments (e.g., manganese violet, cobalt violet, etc.); inorganic green pigments (e.g., chromium oxide, chromium hydroxide, cobalt titanate, etc.); inorganic blue pigments (e.g., ultramarine, Prussian blue, etc.); pearl pigments (e.g., titanium oxide-coated mica, titanium oxide-coated bismuth oxychloride, titanium oxide-coated talc, colored titanium oxide-coated mica, bismuth oxychloride, fish scale foils, etc.); metal powder pigments (e.g., aluminum powders, copper powders, etc.); organic pigments of zirconium, barium, or aluminum lakes (e.g., organic pigments, such as Red. No. 201, Red. No. 202, Red. No. 204, Red. No. 205, Red. No. 220, Red. No. 226, Red. No. 228, Red. No. 405, Orange No. 203, Orange No. 204, Yellow No. 205, Yellow No. 401, Blue No. 404, and the like; Red. No. 3, Red. No. 104, Red. No. 106, Red. No. 227, Red. No. 230, Red. No. 401, Red. No. 505, Orange No. 205, Yellow No. 4, Yellow No. 5, Yellow No. 202, Yellow No. 203, Green No. 3, Blue No. 1, etc.); natural dyes (e.g., chlorophyll, β-carotene, etc.); and the like.

Examples of the liquid oils and fats include avocado oil, camellia oil, turtle oil, macadamia nut oil, corn oil, mink oil, olive oil, rapeseed oil, egg yolk oil, sesame oil, persic oil, wheat germ oil, camellia kissi seed oil, castor oil, linseed oil, safflower oil, cottonseed oil, perilla oil, soybean oil, peanut oil, tea seed oil, Torreya nucifera seed oil, rice bran oil, China wood oil, Japanese tung oil, jojoba oil, germ oil, triglycerin, and the like.

Examples of the solid oils and fats include cocoa butter, coconut oil, horse fat, hydrogenated coconut oil, palm oil, tallow, mutton tallow, hydrogenated beef tallow, palm kernel oil, lard, beef bone fat, Japan wax kernel oil, hydrogenated oil, neatsfoot oil, Japan wax, hydrogenated castor oil, and the like.

Examples of the wax include beeswax, candelilla wax, cotton wax, carnauba wax, bayberry wax, insect wax, spermaceti wax, montan wax, bran wax, lanolin, Ceiba pentandra wax, lanolin acetate, liquid lanolin, sugarcane wax, lanolin fatty acid isopropyl ester, hexyl laurate, reduced lanolin, jojoba wax, hard lanolin, shellac wax, POE lanolin alcohol ether, POE lanolin alcohol acetate, POE cholesterol ether, polyethylene glycol lanolin fatty acids, POE hydrogenated lanolin alcohol ether, and the like.

Examples of the hydrocarbon oil include liquid paraffin, ozokerite, squalane, pristane, paraffin, ceresin, squalene, Vaseline, microcrystalline wax, and the like.

Examples of the higher fatty acids include lauric acid, myristic acid, palmitic acid, stearic acid, behenic acid, oleic acid, undecylenic acid, tall oil acid, isostearic acid, linoleic acid, linolenic acid, eicosapentaenoic acid (EPA), docosahexaenoic acid (DHA), and the like.

Examples of the higher alcohols include straight-chain alcohols (e.g., lauryl alcohol, cetyl alcohol, stearyl alcohol, behenyl alcohol, myristyl alcohol, oleyl alcohol, cetostearyl alcohol, etc.); branched-chain alcohols (e.g., monostearyl glycerin ether (batyl alcohol), 2-decyltetradecynol, lanolin alcohol, cholesterol, phytosterol, hexyldodecanol, isostearyl alcohol, octyl dodecanol, etc.), and the like.

Examples of the synthetic ester oil include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkylglycol monoisostearate, neopentylglycol dicaprate, diisostearyl malate, glycerin di-2-heptylundecanoate, trimethylolpropane tri-2-ethylhexanoate, trimethylolpropane triisostearate, pentaerythritol tetra-2-ethylhexanoate, glycerin tri-2-ethylhexanoate, glycerin trioctanoate, glycerin triisopalmitate, trimethylolpropane triisostearate, cetyl 2-ethylhexanoate, 2-ethylhexyl palmitate, glycerin trimyristate, glyceride tri-2-heptylundecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, and the like.

Examples of the silicone oil include chain polysiloxane (e.g., dimethyl polysiloxane, methylphenyl polysiloxane, diphenyl polysiloxane, etc.); cyclic polysiloxane (e.g., octamethylcyclotetrasiloxane, decamethylcyclopentasiloxane, dodecamethylcyclohexasiloxane, etc.), silicone resins forming a three-dimensional network structure, silicone rubber, various modified-polysiloxane (amino-modified polysiloxane, polyether-modified polysiloxane, alkyl-modified polysiloxane, fluorine-modified polysiloxane, etc.), and the like.

Examples of the silicones include: alkyl and alkoxy silicone wax, such as stearyl-modified dimethicone, behenyl-modified dimethicone, and the like; silicone resins forming a three-dimensional network structure; silicone rubber; and the like.

Examples of the anionic surfactant include: fatty acid soaps (e.g., sodium laurate, sodium palmitate, etc.); higher alkyl sulfates (e.g., sodium lauryl sulfate, potassium lauryl sulfate, etc.); alkyl ether sulfates (e.g., triethanolamine POE lauryl ether sulfate, sodium lauryl POE sulfate, etc.); N-acylsarcosine acid (e.g., sodium lauroylsarcosine, etc.); higher fatty acid amide sulfonic acid (e.g., sodium N-myristoryl-N-methyltaurate, sodium cocoyl methyl taurate, sodium lauryl methyl taurate, etc.); phosphate salts (e.g., sodium POE oleyl ether phosphate, POE-stearyl ether phosphate, etc.); sulfosuccinate salts (e.g., sodium di-2-ethylhexyl sulfosuccinate, sodium mono(lauroylethanolamide polyoxyethylene) sulfosuccinate, sodium lauryl polypropylene glycol sulfosuccinate, etc.); alkyl benzene sulfonates (e.g., sodium linear decyl benzene sulfonate, triethanolamine linear dodecyl benzene sulfonate, linear dodecyl benzene sulfonate, etc.); higher fatty acid ester sulfate salts (e.g., sodium hydrogenated glyceryl cocoate sulfate, etc.); N-acyl glutamate (e.g., monosodium N-lauroyl glutamate, disodium N-stearoyl glutamate, monosodium N-myristoyl-L-glutamate, etc.); sulfated oil (e.g., sulfated castor oil, etc.); POE alkyl ether carboxylic acid; POE alkyl allyl ether carboxylate; α-olefin sulfonate; higher fatty acid ester sulfonate; secondary alcohol sulfate; higher fatty acid alkylolamide sulfate salts; sodium lauloyl monoethanolamide succinate; di-triethanolamine N-palmitoylaspartate; casein sodium; and the like.

Examples of the cationic surfactants include alkyl trimethyl ammonium salts (e.g., stearyl trimethyl ammonium chloride, lauryl trimethyl ammonium chloride, etc.); alkyl pyridinium salts (e.g., cetyl pyridinium chloride, etc.); distearyl dimethyl ammonium dialkyl dimethyl ammonium chloride; poly(N,N'-dimethyl-3,5-methylenepiperidinium) chloride; alkyl quaternary ammonium salts; alkyl dimethylbenzyl ammonium salts; alkylisoquinolinium salts; dialkylmorpholinium salts; POE-alkylamine; alkylamine salts; polyamine fatty acid derivatives; amyl alcohol fatty acid derivatives; benzalkonium chloride; benzethonium chloride; and the like.

Examples of the amphoteric surfactant include: imidazoline-based amphoteric surfactants (e.g., sodium 2-undecyl-N,N,N-(hydroxyethylcarboxymethyl)-2-imidazoline, 2-cocoyl-2-imidazolinium hydroxide-1-carboxyethyloxy disodium salt, etc.); betaine-based surfactants (e.g., 2-heptadecyl-N-carboxymethyl-N-hydroxyethylimidazolinium betaine, lauryl dimethylaminoacetic acid betaine, alkyl betaine, amidobetaine, sulfobetaine, etc.); and the like.

Examples of the lipophilic nonionic surfactant include: sorbitan fatty acid esters (e.g., sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, diglycerol sorbitan tetra-2-ethylhexylate, etc.); glycerin polyglycerin fatty acids (e.g., cottonseed oil monoglyceride, glycerin monoerucate, glyceryl sesquioleate, glycerin monostearate, glyceryl α,α'-oleate pyrrolidone carboxylate, glyceryl monostearate malate, etc.); propylene glycol fatty acid esters (e.g., propylene glycol monostearate, etc.); hydrogenated castor oil derivatives; glycerin alkyl ethers; and the like.

Examples of the hydrophilic nonionic surfactants include: POE-sorbitan fatty acid esters (e.g., POE-sorbitan monooleate, POE-sorbitan monostearate, POE-sorbitan monooleate, POE-sorbitan tetraoleate, etc.); POE sorbitol fatty acid esters (e.g., POE-sorbitol monolaurate, POE-sorbitol monooleate, POE-sorbitol pentaoleate, POE-sorbitol monostearate, etc.); POE-glycerin fatty acid esters (e.g., POE-monooleates, such as POE-glycerol monostearate, POE-glycerol monoisostearate, POE-glycerol triisostearate, etc.); POE-fatty acid esters (e.g., POE-distearate, POE-monodioleate, ethylene glycol distearate, etc.); POE-alkyl ethers (e.g., POE-lauryl ether, POE-oleyl ether, POE-stearyl ether, POE-behenyl ether, POE-2-octyldodecyl ether, POE-cholestanol ether, etc.); Pluronic (registered trademark) types (e.g., Pluronic (registered trademark), etc.); POE·POP-alkyl ethers (e.g., POE·POP-cetyl ether, POE·POP-2-decyltetradecyl ether, POE·POP-monobutyl ether, POE·POP-hydrogenated lanolin, POE·POP-glycerin ether, etc.); tetra-POE·tetra-POP-ethylene diamine condensates (e.g., Tetronic, etc.);POE-castor oil hydrogenated castor oil derivatives (e.g., POE-castor oil, POE-hydrogenated castor oil, POE-hydrogenated castor oil monoisostearate, POE-hydrogenated castor oil triisostearate, POE-hydrogenated castor oil monopyroglutamic acid isostearic acid diester, POE-hydrogenated castor oil maleic acid, etc.); POE-beeswax lanolin derivatives (e.g., POE-sorbitol beeswax, etc.); alkanolamides (e.g., coconut oil fatty acid diethanolamide, lauric acid monoethanolamide, fatty acid isopropanolamide, etc.); POE-propylene glycol fatty acid esters; POE-alkylamines; POE-fatty acid amides; sucrose fatty acid esters; alkylethoxydimethylamine oxide; trioleyl phosphate; and the like.

Examples of the active agents other than the surfactants include active agents that are emulsified by fine particles. Specific examples of the active agents other than the surfactants include active agents for Pickering emulsions, and the like. Examples of the active agents for Pickering emulsions include (acrylamide/DMAPA acrylate/methoxy PEG methacrylate) copolymers, hydrophobic silica, and the like. Since the (acrylamide/DMAPA acrylate/methoxy PEG methacrylate) copolymer has water resistance, the (acrylamide/DMAPA acrylate/methoxy PEG methacrylate) copolymer is suitably used when the oil-in-water emulsion cosmetic is desired to have water resistance.

In the case of the active agent that is emulsified by fine particles, it is assumed that the amphiphile is inserted into the gaps between the fine particles, thereby enhancing emulsion stability.

In addition, in the case where the oil-in-water emulsion cosmetic of the present invention includes the active agent that is emulsified by fine particles and the polyoxyethylene hydrogenated castor oil, it is assumed that, since the polyoxyethylene hydrogenated castor oil is a surfactant having a relatively small molecular weight, emulsion stability of the oil-in-water emulsion cosmetic can be improved by use of the active agent that is emulsified by fine particles, such as the active agent for Pickering emulsions, or the like, in combination.

Examples of the natural water-soluble polymers include: plant-based polymers (e.g., gum arabic, tragacanth gum, galactan, guar gum, carob gum, karaya gum, carrageenan, pectin, agar, quince seed (quince), algae colloid (blown algae extract), starches (rice, corn, potato, and wheat), and glycyrrhizic acid); microorganism-based polymers (e.g., xanthan gum, dextran, succinoglycan, pullulan, etc.); and the like.

Examples of the semisynthetic water-soluble polymers include: starch-based polymers (e.g., carboxymethyl starch, methylhydroxypropyl starch, etc.); cellulose-based polymers (e.g., methyl cellulose, ethyl cellulose, methyl hydroxypropyl cellulose, hydroxyethyl cellulose, sodium cellulose sulfate, hydroxypropyl cellulose, carboxymethyl cellulose, sodium carboxymethyl cellulose, crystalline cellulose, cellulose powders, etc.); alginic acid-based polymers (e.g., sodium alginate, propylene glycol alginate, etc.); and the like.

Examples of the synthetic water-soluble polymers include: vinyl polymers (e.g., polyvinyl alcohol, polyvinyl methyl ether, polyvinyl pyrrolidone, carboxyvinyl polymers, etc.); polyoxyethylene-based polymers (e.g., polyoxyethylene polyoxypropylene copolymers of polyethylene glycol 20,000, 40,000, or 60,000, etc.); acrylic polymers (e.g., sodium polyacrylate, polyethyl acrylate, polyacrylamide, etc.); polyethyleneimine; cationic polymers; and the like.

Examples of the thickening agents include gum arabic, carrageenan, karaya gum, tragacanth gum, carob gum, quince seed (quince), casein, dextrin, gelatin, sodium pectinate, sodium alaginate, methyl cellulose, ethyl cellulose, CMC, hydroxyethyl cellulose, hydroxypropyl cellulose, PVA, PVM, PVP, sodium polyacrylate, carboxyvinyl polymers, locust bean gum, guar gum, tamarind gum, dialkyldimethyl ammonium sulfate cellulose, xanthan gum, aluminum magnesium silicate, bentonite, hectorite, Al Mg silicate (veegum), laponite, silicic anhydride, carbomers, (dimethylacrylamide/Na acryloyldimethyltaurate) crosspolymers, (Na acrylate/Na acryloyldimethyltaurate) copolymers, (ammonium acryloyldimethyltaurate/beheneth-25 methacrylate) crosspolymers, (ammonium acryloyldimethyltaurate/VP) copolymers, (hydroxyethyl acrylate/Na acryloyldimethyltaurate) copolymers, (acrylate/alkyl (C10-30) acrylate) crosspolymers, and the like. Note that the (acrylate/alkyl (C10-30) acrylate) crosspolymers also function as an active agent or an emulsifier.

Examples of the ultraviolet-ray absorbers include: benzoic acid-based ultraviolet-ray absorbers (e.g., para-aminobenzoic acid (hereinafter, abbreviated as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA ethyl ester, etc.); anthranilic acid-based ultraviolet-ray absorbers (e.g., homomenthyl-N-acetylanthranilate, etc.); salicylic acid-based ultraviolet-ray absorbers (e.g., amyl salicylate, menthyl salicylate, homomenthyl salicylate, octyl salicylate, phenyl salicylate, benzyl salicylate, p-isopropanolphenyl salicylate, etc.); cinnamic acid-based ultraviolet-ray absorbers (e.g., octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate, glyceryl mono-2-ethylhexanoyl-diparamethoxy cinnamate, etc.); benzophenone-based ultraviolet-ray absorbers (e.g., 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone, etc.); 3-(4'-methylbenzylidene)-d,l-camphor, 3-benzylidene-d,l- camphor; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenylbenzotriazole; dibenzalazine; dianisoylmethane; 4-methoxy-4'-t-butyldibenzoylmethane; 5-(3,3-dimethyl-2-norbornylidene)-3-pentan-2-one; and the like.

Examples of the moisturizing agent include glycerin, dynamite glycerin, dipropylene glycol, 1,3-butylene glycol, cholesteryl-12-hydroxystearate, sodium lactate, dl-pyrrolidone carboxylate, urea, diglycerin ethylene oxide·propylene oxide adducts, and the like. In particular, when acidic mucopolysaccharide, such as chondroitin sulfate, hyaluronic acid, or the like is added, the moisturizing agent has an effect of enhancing a whitening effect of alkoxysalicylic acid, a salt of alkoxysalicylic acid, or both.

Examples of the sequestering agents include 1-hydroxyethane-1,1-diphosphonic acid, tetrasodium 1-hydroxyethane-1,1-diphosphonate, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, trisodium

### ethylenediaminehydroxyethyltriacetate, and the like.

Examples of the lower alcohols include ethanol, propanol, isopropanol, isobutyl alcohol, t-butyl alcohol, and the like.

Examples of the polyhydric alcohols include dihydric alcohols (e.g., ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol, etc.); trihydric alcohols (e.g., glycerin, trimethylol propane, etc.); tetrahydric alcohols (e.g., pentaerythritol, such as 1,2,6-hexanetriol, etc.); pentahydric alcohols (e.g., xylitol, etc.); hexahydric alcohols (e.g., sorbitol, mannitol, etc.); polyhydric alcohol polymers (e.g., diethylene glycol, dipropylene glycol, triethylene glycol, polypropylene glycol, tetraethylene glycol, diglycerin, polyethylene glycol, triglycerin, tetraglycerin, polyglycerin, etc.); dihydric alcohol alkyl ethers (e.g., ethylene glycol monomethyl ether, ethylene glycol monoethyl ether, ethylene glycol monobutyl ether, ethylene glycol monophenyl ether, ethylene glycol monohexyl ether, ethylene glycol mono(2-methylhexyl) ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether, etc.); dihydric alcohol alkyl ethers (e.g., diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether, etc.); dihydric alcohol ether esters (e.g., ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate, etc.); glycerin monoalkyl ether (e.g., xyl alcohol, selachyl alcohol, batyl alcohol, etc.); sugar alcohols (e.g., sorbitol, maltitol, maltotriose, mannitol, sucrose, erythritol, glucose, fructose, starch hydrolysate, maltose, xylitol, hydrogenated starch hydrolysate, etc.); glysolid; tetrahydrofurfuryl alcohol; POE-tetrahydrofurfuryl alcohol; POP-butyl ether; POP·POE-butyl ether; tri(polyoxy propylene) glycerin ether; POP-glycerin ether; POP-glycerol ether phosphate; POP·POE-pentaerythritol ether; polyglycerin; and the like.

Examples of the monosaccharides include trioses (e.g., D-glycerylaldehyde, dihydroxyacetone, etc.); tetraoses (e.g., D-erythrose, D-erythrulose, D-threose, erythrytol, etc.); pentoses (e.g., L-arabinose, D-xylose, L-lyxose, D-arabinose, D-ribose, D-ribulose, D-xylulose, L-xylulose, etc.); hexoses (e.g., D-glucose, D-talose, D-bucyclose, D-galactose, D-fructose, L-galactose, L-mannose, D-tagatose, etc.); heptoses (e.g., aldoheptose, heptulose, etc.); octoses (e.g., octulose, etc.); deoxysaccharides (e.g., 2-deoxy-D-ribose, 6-deoxy-L-galactose, 6-deoxy-L-mannose, etc.); aminosugars (e.g., D-glucosamine, D-galactosamine, sialic acid, aminouronic acid, muramic acid, etc.); uronic acid (e.g., D-glucuronic acid, D-mannuronic acid, L-guluronic acid, D-galacturonic acid, L-iduronic acid, etc.); and the like.

Examples of the oligosaccharide include sucrose, gentianose, umbelliferose, lactose, planteose, isolyxoses, α,α-trehalose, raffinose, lyxoses, umbilysin, stachyose verbascose, and the like.

Examples of the polysaccharide include cellulose, quince seed, chondroitin sulfate, starch, galactan, dermatan sulfate, glycogen, gum arabic, heparan sulfate, hyaluronic acid, tragacanth gum, keratan sulfate, chondroitin, xanthan gum, mucoitin sulfate, guar gum, dextran, keratosulfate, locust bean gum, succinoglucan, caronic acid, and the like.

Examples of the amino acids include: neutral amino acids (e.g., threonine, cysteine, etc.); basic amino acids (e.g., hydroxylysine, etc.); and the like. Examples of the amino acid derivatives include sodium acyl sarcosine (sodium lauroyl sarcosine), acyl glutamate, sodium acyl β-alanine, glutathione, pyrrolidone carboxylic acid, and the like.

Examples of the organic amines include monoethanolamine, diethanolamine, triethanolamine, morpholine, triisopropanolamine, 2-amino-2-methyl-1,3-propanediol, 2-amino-2-methyl-1-propanol, and the like.

Examples of the polymer emulsion include acrylic resin emulsions, ethyl polyacrylate emulsions, fluid acryl resins, polyacrylic alkyl ester emulsions, polyvinyl acetate resin emulsions, natural rubber latex, and the like.

Examples of the neutralizing agents include: inorganic bases, such as sodium hydroxide, potassium hydroxide, and the like; and organic amines, such as monoethanolamine, diethanolamine, triethanolamine, morpholine, triisopropanolamine, 2-amino-2-methyl-1,3-propanediol, 2-amino-2-methyl-1-propanol, and the like.

Examples of the pH adjusters include buffers, such as lactic acid-sodium lactate, citric acid-sodium citrate, succinic acid-sodium succinate, and the like.

Examples of the vitamins includes: vitamins A, B1, B2, B6, C, and E, and derivatives of the foregoing; panthothenic acid and derivatives of panthothenic acid; biotin; and the like.

Examples of the antioxidants include tocopherols, dibutylhydroxytoluene, butylhydroxyanisole, gallic acid esters, and the like.

Examples of the antioxidation auxiliary agents include phosphoric acid, citric acid, ascorbic acid, maleic acid, malonic acid, succinic acid, fumaric acid, cephalin, hexametaphosphate, phytic acid, ethylenediaminetetraacetic acid, and the like.

Examples of the stabilizers include Na pyrosulfite, EDTA-2Na, and the like.

Examples of other components that can be blended include: preservatives (phenoxyethanol, ethyl paraben, butyl paraben, chlorphenesine, etc.); anti-inflammatory agents (e.g., glycyrrhizic acid derivatives, glycyrrhetinic acid derivatives, salicylic derivatives, hinokitiol, zinc oxide, allantoin, etc.); whitening agents (e.g., placental extracts, extracts of Saxifraga L., vitamin C, magnesium ascorbate phosphate, ascorbic acid glucoside, arbutin, alkoxysalicylic acid, salts of the foregoing, etc.); various extracts (e.g., phellodendron bark, coptis rhizome, lithospermum root, peony, swertia herb, birch, sage, loquat, carrots, aloe vera, common mallow, iris, grapes, coix seed, luffa, lily, saffron, cnidium rhizome, ginger, St. John's wort, restharrow, garlic, capsicum plants, citrus unshiu peel, Angelica acutiloba, seaweeds, etc.), activators (e.g., royal jelly, photosensitizers, cholesterol derivatives, etc.); blood circulation promoters (e.g., nonanic acid vanillylamide, benzyl nicotinate, β-butoxyethyl nicotinate, capsaicin, zingerone, Cantharides tincture, ichthamol, tannic acid, α-borneol, tocopherol nicotinate, inositol hexanicotinate, cyclandelate, cinnarizine, tolazoline, acetylcholine, verapamil, cefalantine, γ-oryzanol, etc.); anti-seborrheic agents (e.g., sulfur, thianthol, etc.); anti-inflammatory agents (e.g., tranexamic acid, thiotaurine, hypotaurine, etc.); and the like.

The oil-in-water emulsion cosmetic according to the present invention is suitably used in various dosage forms, such as creams, milky lotions, fluids, and the like. As a product form of the oil-in-water emulsion cosmetic, skin care cosmetics, such as sunscreens, and the like, or make-up cosmetics, such as make-up base, foundations, and the like, can be used.

### Examples

Hereinafter, the present invention will be described in detail with reference to Examples, but the present invention is not limited to Examples below. Unless otherwise specified, all blended amounts are represented in percent by mass relative to a total amount.

Amphiphiles 1 to 5 and Comparative Substances 1 to 4 below were prepared.

### (Amphiphile 1)

PEG/PPG-9/2 dimethyl ether
(General formula (1) in which R¹: CH₃, R²: CH₃, AO: CH(CH₃)CH₂O, EO: CH₂CH₂O, m: 2, and n: 9)

### (Amphiphile 2)

PEG/PPG-14/7 dimethyl ether
(General formula (1) in which R¹: CH₃, R²: CH₃, AO: CH(CH₃)CH₂O, EO: CH₂CH₂O, m: 7, and n: 14)

### (Amphiphile 3)

PEG/PPG-36/41 dimethyl ether
(General formula (1) in which R¹: CH₃, R²: CH₃, AO: CH(CH₃)CH₂O, EO: CH₂CH₂O, m: 41, and n: 36)

### (Amphiphile 4)

PEG/PPG-17/4 dimethyl ether
(General formula (1) in which R¹: CH₃, R²: CH₃, AO: CH(CH₃)CH₂O, EO: CH₂CH₂O, m: 4, and n: 17)

### (Amphiphiles 5 and 6)

Amphiphiles 5 and 6 were polymers represented by the general formula (2), in which A of the general formula (2) was an alkylene group represented by the general formula (3).

The structure of Amphiphile 5 was described as Synthesis Example 1 in Table 1. The structure of Amphiphile 6 was as described as Synthesis Example 2 in Table 1. Amphiphiles 5 and 6 were synthesized by the following methods.

### <Synthesis of Amphiphile 5>

An autoclave was loaded with 128 g of methanol and 6.4 g of potassium hydroxide serving as a catalyst. After replacing the air inside the autoclave with dry nitrogen, the catalyst was completely dissolved at 80°C with stirring. Next, a mixture of 592 g of glycidol and 1,392 g of propylene oxide was added dropwise at 95°C over 20 hours by a dripping device, followed by stirring for 5 hours. The resultant reaction composition was taken out from the autoclave, was neutralized with hydrochloric acid to pH 6 to 7, and was processed under reduced pressure of -0.095 MPa (50 mmHg) at 100°C for 1 hour to remove the water included in the reaction composition. After the processing, filtration was further performed to remove the generated salts, thereby yielding 2,000 g of Amphiphile 5.

### <Synthesis of Amphiphile 6>

An autoclave was loaded with 128 g of methanol and 6.4 g of potassium hydroxide serving as a catalyst. After replacing the air inside the autoclave with dry nitrogen, the catalyst was completely dissolved at 80°C with stirring. Next, a mixture of 592 g of glycidol and 1,392 g of propylene oxide was added dropwise at 95°C over 20 hours by a dripping device, followed by stirring for 2 hours. Next, 244 g of potassium hydroxide was added. After purging the reaction system with dry nitrogen, 200 g of methyl chloride was added under the pressure and allowed to react at a temperature of 80 to 130°C for 5 hours. The resultant reaction composition was taken out from the autoclave, was neutralized with hydrochloric acid to pH 6 to 7, and was processed under reduced pressure of -0.095 MPa (50 mmHg) at 100°C for 1 hour to remove the water included in the reaction composition. After the processing, filtration was further performed to remove the generated salts, thereby yielding 1,820 g of Amphiphile 6.

### <Number average molecular weights of Amphiphiles 5 and 6>

The number average molecular weights of Amphiphiles 5 and 6 were measured by the following method.

The number average molecular weights of Amphiphiles 5 and 6 were determined by gel permeation chromatography (GPC) measurement. A SHODEX (registered trademark) GPC101GPC dedicated system serving as a system, SHODEX (registered trademark) RI-71s serving as a differential refractometer, SHODEX (registered trademark) KF-G serving as a guard column, and three-coupled SHODEX (registered trademark) KF804L serving as columns were installed. Tetrahydrofuran serving as a developing solution was allowed flow at a flow rate of 1 mL/min at the column temperature of 40°C, and 0.1 mL of a 0.1 wt% tetrahydrofuran solution of the obtained reaction product was injected, thereby obtaining, using a BORWINGPC calculation program, a chromatogram represented by the refractive intensity and the elution time. The number average molecular weight was determined from the chromatogram using polyethylene glycol as a standard.

The number average molecular weights of Amphiphiles 5 and 6 were as presented in Table 1.

### <IOB values of Amphiphiles 5 and 6>

The IOB values of Amphiphiles 5 and 6 were determined by the method described in "Organic Conceptual Diagram-Foundation and Application-", p. 11-17 (Yoshio KODA, SANKYO SHUPPAN Co., Ltd., published in 1984).

The IOB values of Amphiphiles 5 and 6 were as presented in Table 1.

**[Table 1]**

| Synthesis Example No. (Polymer No.) | 1 | 2 |
|---|---|---|
| General Formula (2) (A in General Formula (2) is General Formula (3)) | 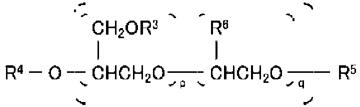 | |
| R³ | H | CH₃ and H |
| R⁴ | H | CH₃ and H |
| R⁵ | CH₃ | CH₃ |
| R⁶ | CH₃ | CH₃ |
| Ratio of CH₃ and H in R³ and R⁴(CH₃/H) | - | 0.57 |
| p | 2.0 | 2.0 |
| q | 6.0 | 6.0 |
| p+q | 8.0 | 8.0 |
| Polymerization form | Random | Random |
| Number average molecular weight | 530 | 673 |
| Weight average molecular weight | 859 | 956 |
| Polydispersity index (Mw/Mn) | 1.62 | 1.42 |
| IOB value | 1.09 | 0.77 |
| Hydroxyl group capping rate (%) | - | 57 |
| Hydroxyl value (mgKOH/g) | 294 | 125 |
| Cloud point (5%aq.) | 74°C | 45°C |
| Solubility (water) | 50 mass% or greater | 30 mass% or greater |
| Solubility (oil) *1 | ○ | ○ |

| | | |
|---|---|---|
| *1 Synthesized compound was mixed with olive oil at the concentration of 1.0 percent by mass, and the appearance of the mixture was checked. When the appearance of the mixture was homogeneous, it was evaluated as "○". | | |

### (Comparative Substance 1)

### Bisethoxy diglycol cyclohexane-1,4-dicarboxylate

### (Comparative Substance 2)

### Di(phytosteryl/octyldodecyl) lauroyl glutamate (Eldew PS203, Ajinomoto Co., Inc.)

### (Comparative Substance 3)

### PEG-20 (PEG#1000)

### (Comparative Substance 4)

### Diethoxyethyl succinate (CRODAMOL DES, Croda Japan K. K.)

Note that diethoxyethyl succinate is the compound represented by the following formula.

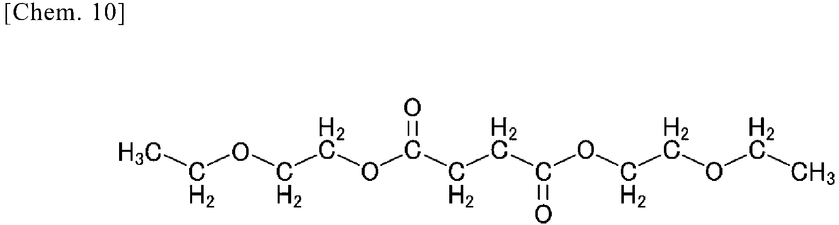

### [Examples 1 to 7 and Comparative Examples 1 to 6]

Cosmetics of Examples 1 to 7 and Comparative Examples 1 to 6 were prepared using Amphiphiles 1 to 6 and Comparative Substances 1 to 4 according to the compositions presented in Tables 2 and 3, and emulsion stability of each oil-in-water emulsion cosmetic was evaluated by the following method.

### [Emulsion stability]

A tube was filled with the produced oil-in-water emulsion cosmetic, followed by compressing the tube 1,000 times or more. The presence or absence of oil leaching due to demulsification was visually observed, and emulsion stability was evaluated based on the following evaluation criteria. When demulsification occurs, leaching of oil is observed.

### (Evaluation criteria)

A: The appearance was homogeneous and no oil leaching was observed.
B: Oil leaching was partially observed.
C: Oil leaching was observed over a large area.

The oil-in-water emulsion cosmetic evaluated as "A" or "B" according to the above evaluation criteria was determined to be practicable as the present invention.

**[Table 2]**

| Oil-in-water emulsion cosmetic | | Ex. 1 | Ex. 2 | Ex. 3 | Ex. 4 | Ex. 5 | Ex. 6 | Ex. 7 |
|---|---|---|---|---|---|---|---|---|
| Amphiphile 1 | PEG/PPG-9/2 dimethyl ether | 3 | 3 | | | | | |
| Amphiphile 2 | PEG/PPG-14/7 dimethyl ether | | | 3 | | | | |
| Amphiphile 3 | PEG/PPG-36/41 dimethyl ether | | | | 3 | | | |
| Amphiphile 4 | PEG/PPG-17/4 dimethyl ether | | | | | 3 | | |
| Amphiphile 5 | Polymer of Synthesis Example 1 | | | | | | 3 | |
| Amphiphile 6 | Polymer of Synthesis Example 2 | | | | | | | 3 |
| Metal oxide fine particles | Cetyl phosphate-treated silica-cated titanium oxide (average primary particle diameter: 15nm) | 4 | 4 | 4 | 4 | 4 | 4 | 4 |
| Active agent | PEG-60 hydrogenated castor oil | 1.5 | 3 | 1.5 | 1.5 | 1.5 | 1.5 | 1.5 |
| Oil phase | Diisopropyl sebacate | 10 | 10 | 10 | 10 | 10 | 10 | 10 |
| | Ethylhexyl methoxycnnamate | 10 | 10 | 10 | 10 | 10 | 10 | 10 |
| | Polyglyceryl-6 polyricinoleate | 0.5 | 0.5 | 0.5 | 0.5 | 0.5 | 0.5 | 0.5 |
| | Dimethicone (6cs) | 3 | 3 | 3 | 3 | 3 | 3 | 3 |
| | Isododecane | 3 | 3 | 3 | 3 | 3 | 3 | 3 |
| Water phase | (Acrylate/alkyl(C10-30) acrylate) crosspolymer | 0.08 | 0.08 | 0.08 | 0.08 | 0.08 | 0.08 | 0.08 |
| | Carbomer | 0.22 | 0.22 | 0.22 | 0.22 | 0.22 | 0.22 | 0.22 |
| | Xanthan gum | 0.1 | 0.1 | 0.1 | 0.1 | 0.1 | 0.1 | 0.1 |
| | Glycerin | 2 | 2 | 2 | 2 | 2 | 2 | 2 |
| | Ethanol | 7 | 7 | 7 | 7 | 7 | 7 | 7 |
| | Silica (2-10*µ*m) | 1.2 | 1.2 | 1.2 | 1.2 | 1.2 | 1.2 | 1.2 |
| | (Acrylamide/DMAPA acrylate/methoxy PEG methacrylate) copolymer (purity:10%) | 10 | | 10 | 10 | 10 | 10 | 10 |
| | (Dimethylacrylamide/Na acryloyldimethyltaurate) crosspolymer | | 0.2 | | | | | |
| | K hydroxide | 0.3 | 0.3 | 0.3 | 0.3 | 0.3 | 0.3 | 0.3 |
| | Water | balance | balance | balance | balance | balance | balance | balance |
| Total | | 100 | 100 | 100 | 100 | 100 | 100 | 100 |
| Emulsion stability | | A | A | A | A | B | B | B |

| Oil-in-water emulsion cosmetic | | Comp. Ex. 1 | Comp. Ex. 2 | Comp. Ex. 3 | Comp. Ex. 4 | Comp. Ex. 5 | Comp. Ex. 6 |
|---|---|---|---|---|---|---|---|
| Comparative substance 1 | Bis-ethoxydiglycol cyclohexane 1,4-dicarboxylate | | | 3 | | | |
| Comparative substance 2 | Di(phytosteryl/octyldodecyl) lauroyl glutamate | | | | 3 | | |
| Comp. subs. 3 | PEG-20 | | | | | 3 | |
| Comp. subs. 4 | Diethoxyethyl succinate | | | | | | 3 |
| Metal oxide fine particles | Cetyl phosphate-treated silica-coated titanium oxide (average primary particle diameter: 15nm) | 4 | 4 | 4 | 4 | 4 | 4 |
| Active agent | PEG-60 hydrogenated castor oil | 1.5 | 3 | 1.5 | 1.5 | 1.5 | 1.5 |
| Oil phase | Diisopropyl sebacate | 10 | 10 | 10 | 10 | 10 | 10 |
| | Ethylhexyl methoxycinnamate | 10 | 10 | 10 | 10 | 10 | 10 |
| | Polyglyceryl-6 polyricinoleate | 0.5 | 0.5 | 0.5 | 0.5 | 0.5 | 0.5 |
| | Dimethicone (6cs) | 3 | 3 | 3 | 3 | 3 | 3 |
| | Isododecane | 3 | 3 | 3 | 3 | 3 | 3 |
| Water phase | (Acrylate/alkyl(C10-30) acrylate) crosspolymer | 0.08 | 0.08 | 0.08 | 0.08 | 0.08 | 0.08 |
| | Carbomer | 0.22 | 0.22 | 0.22 | 0.22 | 0.22 | 0.22 |
| | Xanthan gum | 0.1 | 0.1 | 0.1 | 0.1 | 0.1 | 0.1 |
| | Glycerin | 2 | 2 | 2 | 2 | 2 | 2 |
| | Ethanol | 7 | 7 | 7 | 7 | 7 | 7 |
| | Silica (2-10 *µ*m) | 1.2 | 1.2 | 1.2 | 1.2 | 1.2 | 1.2 |
| | (Acrylamide/DMAPA acrylate/methoxy PEG methacrylate) copolymer (purity: 10%) | 10 | | 10 | 10 | 10 | 10 |
| | (Dimethylacrylamide/ Na acryloyldimethyltaurate) crosspolymer | | 0.2 | | | | |
| | K hydroxide | 0.3 | 0.3 | 0.3 | 0.3 | 0.3 | 0.3 |
| | Ion-exchanged water | balance | balance | balance | balance | balance | balance |
| Total | | 100 | 100 | 100 | 100 | 100 | 100 |
| Emulsion stability | | C | C | C | C | C | C |

All of Examples 1 to 7 had favrable emulsion stability at a level at which it could be practicable as the present invention.

In Comparative Examples 1 to 6, oil leaching was observed over a large area, and the emulsion stability was poor.

As described above, it was demonstrated that the oil-in-water emulsion cosmetic satisfying the configuration of the present invention could be provided as an oil-in-water emulsion cosmetic that was highly safe and had favorable emulsion stability.

As the oil-in-water emulsion cosmetic of the present invention, the oil-in-water emulsion cosmetics having the configurations of embodiments 1 and 2 presented in Table 4 can also be used.

**[Table 4]**

| Oil-in-water emulsion cosmetic | Embodiment 1 | Embodiment 2 |
|---|---|---|
| PEG/PPG-9/2 dimethyl ether | 3 | |
| PEG/PPG-14/7 dimethyl ether | | 3 |
| Cetyl phosphate-treated silica-coated titanium oxide (average primary particle diameter: 15nm) | 4 | 10 |
| PEG-60 hydrogenated castor oil | 0.1 | 0.5 |
| Octocrylene | 5 | 5 |
| t-Butyl methoxybenzoylmethane | 2 | 2 |
| Alkyl benzoate | 4 | 4 |
| Homosalate | 3 | 3 |
| Dimethicone (6cs) | 4 | 4 |
| Batyl alcohol | 0.2 | 1 |
| Stearyl alcohol | 1 | 0.8 |
| Behenyl alcohol | 4 | 2.5 |
| Beheneth-20 | 1 | 1.7 |
| Butylene glycol | 3.5 | 3.5 |
| Silicic anhydride (micro porous, average primary particle diameter: 10*µ*m) | 0.5 | 0.5 |
| Ethanol | 4 | 4 |
| Phenoxy ethanol | 0.5 | 0.5 |
| Citric acid | 0.02 | 0.05 |
| Na Citrate | 0.08 | 0.05 |
| Water | Balance | Balance |
| Total | 100 | 100 |

Embodiments of the present invention include, for example, the following.
<1> An oil-in-water emulsion cosmetic, including:
   (A) an amphiphile; and
   (B) hydrophobic-treated metal oxide fine particles that have been subjected to a hydrophobic treatment using monoalkyl phosphate,

   wherein the (A) amphiphile includes one or more selected from the group consisting of alkylene oxide derivatives represented by a general formula (1) below and a general formula (2) below, 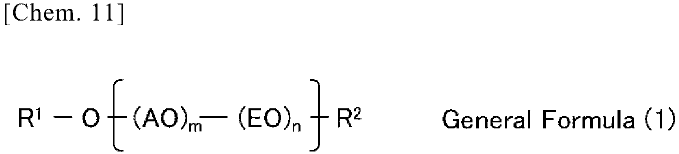 (in the general formula (1), AO is an oxyalkylene group with 3 to 4 carbon atoms, EO is an oxyethylene group, R¹ and R² are each independently a hydrocarbon group with 1 to 4 carbon atoms or a hydrogen atom, and m and n are integers of 1≤ m ≤ 70, and 1 ≤ n ≤ 70, respectively) 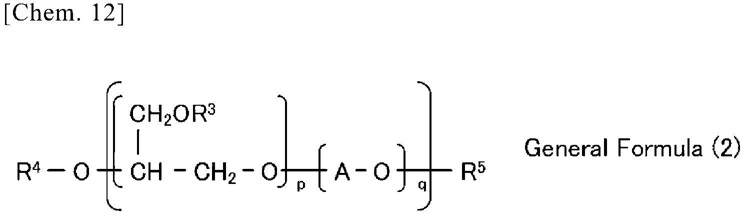 (in the general formula (2), R³, R⁴, and R⁵ are each independently a hydrogen atom or an alkyl group with 1 to 4 carbon atoms, A is an alkylene group with 2 to 4 carbon atoms, and p and q are each independently an integer of 1 to 50, where a polymer represented by the general formula (2) has a number average molecular weight of 10,000 or less and an IOB value of 0.4 to 1.8).
<2> The oil-in-water emulsion cosmetic according to <1>, further including (C) an active agent.
<3> The oil-in-water emulsion cosmetic according to <2>, wherein the (C) active agent includes polyoxyethylene hydrogenated castor oil.
<4> The oil-in-water emulsion cosmetic according to <3>,
   wherein an average number of moles of ethylene oxide added in the polyoxyethylene hydrogenated castor oil is 50 moles or greater and 100 moles or less.
<5> The oil-in-water emulsion cosmetic according to <1>, <2>, <3>, or <4>,
   wherein the (B) hydrophobic-treated metal oxide fine particles that have been subjected to the hydrophobic treatment using monoalkyl phosphate are metal oxide fine particles each having a silica coating film.
<6> The oil-in-water emulsion cosmetic according to <1>, <2>, <3>, <4>, or <5>,
   wherein the monoalkyl phosphate includes cetyl phosphate.

According to the oil-in-water emulsion cosmetic of any of <1> to <6>, the various problems existing in the related art can be solved, and the object of the present invention can be achieved.

The present international application claims priority based on Japanese Patent Application No. 2023-223656, filed with the Japan Patent Office on December 28, 2023, and the entire contents of which are incorporated herein by reference.

## Claims

1. An oil-in-water emulsion cosmetic, comprising:
(A) an amphiphile; and
(B) hydrophobic-treated metal oxide fine particles that have been subjected to a hydrophobic treatment using monoalkyl phosphate,
wherein the (A) amphiphile includes one or more selected from the group consisting of alkylene oxide derivatives represented by a general formula (1) below and a general formula (2) below, 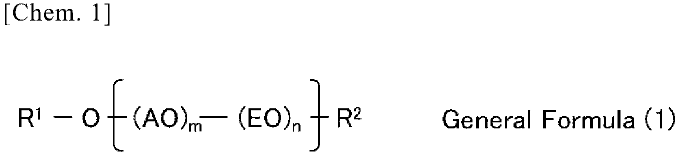 (in the general formula (1), AO is an oxyalkylene group with 3 to 4 carbon atoms, EO is an oxyethylene group, R¹ and R² are each independently a hydrocarbon group with 1 to 4 carbon atoms or a hydrogen atom, and m and n are integers of 1≤ m ≤ 70, and 1 ≤ n ≤ 70, respectively) 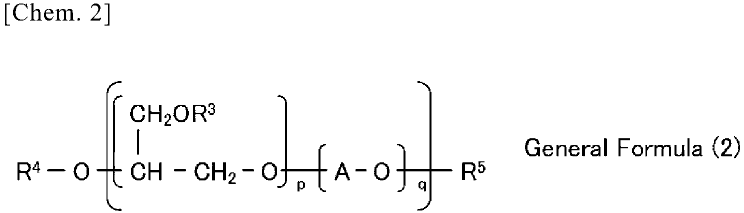 (in the general formula (2), R³, R⁴, and R⁵ are each independently a hydrogen atom or an alkyl group with 1 to 4 carbon atoms, A is an alkylene group with 2 to 4 carbon atoms, and p and q are each independently an integer of 1 to 50, where a polymer represented by the general formula (2) has a number average molecular weight of 10,000 or less and an IOB value of 0.4 to 1.8).

2. The oil-in-water emulsion cosmetic according to claim 1, further comprising:
(C) an active agent.

3. The oil-in-water emulsion cosmetic according to claim 2,
wherein the (C) active agent includes polyoxyethylene hydrogenated castor oil.

4. The oil-in-water emulsion cosmetic according to claim 3,
wherein an average number of moles of ethylene oxide added in the polyoxyethylene hydrogenated castor oil is 50 moles or greater and 100 moles or less.

5. The oil-in-water emulsion cosmetic according to claim 1 or 2,
wherein the (B) hydrophobic-treated metal oxide fine particles that have been subjected to the hydrophobic treatment using monoalkyl phosphate are metal oxide fine particles each having a silica coating film.

6. The oil-in-water emulsion cosmetic according to claim 1 or 2,
wherein the monoalkyl phosphate includes cetyl phosphate.
